# EUROPEAN PATENT APPLICATION

(11) **EP 3 165 198 A1**
(43) Date of publication of application: **10.05.2017**
(21) Application number: 15815480.7
(22) Date of filing: 18.06.2015
(51) Int. Cl.: A61F 2/958, A61F 2/82

(54) **STENT SYSTEM AND METHOD FOR MANUFACTURING SAME**

(30) Priority: 01.07.2014 JP 2014135515
(71) Applicant: Terumo Kabushiki Kaisha, Tokyo 151-0072 (JP)
(72) Inventor: KAWASHIMA, Yoshio, Fujinomiya-shi Shizuoka 418-0015 (JP)
(74) Representative: Casalonga
(86) International application number: PCT/JP2015/067532
(87) International publication number: WO 2016/002526

(57) **Abstract**

A stent system (10) includes a catheter (12) having a balloon (20), and a stent (14) mounted on an outer peripheral portion of the balloon (20). An outer surface of the balloon (20) and an inner surface of the stent (14) have respective modified surfaces, and adhesiveness between the outer surface and the inner surface is improved by the modified surfaces being in contact with each other. A method for manufacturing the stent system (10) includes a surface treatment step of applying surface treatment to each of the outer surface of the balloon (20) and the inner surface of the stent (14) to modify the outer surface and the inner surface in such a way as to improve adhesiveness therebetween.

## Description

### [Technical Field]

The present invention relates to a stent system configured to deliver and place a stent to and at a target site in a living body lumen and to a method for manufacturing the stent system.

### [Background Art]

In recent years, for example, in treatment for acute myocardial infarction or angina pectoris, target vessel revascularization, which is a procedure for improving blood flow by placing a stent at a lesion (stenosed site) of the coronary artery, has been being performed (for example, referring to JP-A-2002-136601). Moreover, stent can be placed for improving a lesion formed in another blood vessel, bile duct, trachea, esophagus, urethra, or other body lumens.

A stent system, which is used to place a stent, usually includes a catheter (deliver catheter) provided with an inflatable and deflatable balloon at the distal portion of a catheter shaft, and a hollow tubular expandable stent mounted on an outer peripheral portion of the balloon. When the balloon is inflated after being delivered to a target site (stenosed site) in the body via a narrow living body lumen, the stent expands while plastically deforming along with inflation of the balloon, so that the stenosed site is widened. After this, when the balloon is deflated, the stent is left in an expanding state, thus being able to keep the stenosed site in a widened state.

Therefore, the stent needs to continue to be stably mounted on an outer peripheral portion of the deflated balloon until the stent reaches the target site in the living body lumen.

During insertion of the stent system into the living body lumen, if the stent comes into contact with, for example, a calcified lesion, the stent may become out of position with or come off the outer peripheral portion of the balloon. In the case where such a situation has occurred, the need for surgically removing the stent may arise, thus causing a decrease in treatment efficiency.

### [Summary of the Invention]

The invention has been made in consideration of such issues, and thus has an object to provide a stent system capable of preventing a stent from becoming out of position with an outer peripheral portion of a balloon and a method for manufacturing the stent system.

To attain the above-mentioned object, the invention is characterized by including a catheter having an inflatable and deflatable balloon, and a stent mounted on an outer peripheral portion of the balloon and configured to expand along with inflation of the balloon, wherein an outer surface of the balloon and an inner surface of the stent have respective modified surfaces, and adhesiveness between the outer surface and the inner surface is improved by the modified surfaces being in contact with each other.

According to the above-mentioned configuration of the invention, since both the outer surface of the balloon and the inner surface of the stent are modified and activated, and the modified surfaces are in contact with each other, the modified surfaces interact with each other, so that adhesiveness between the outer surface of the balloon and the inner surface of the stent can be improved. Accordingly, when the stent is delivered to a lesion in the living body lumen, the stent can be prevented from becoming out of position with the balloon in the living body lumen, or the stent can be prevented from coming off there. Accordingly, the operator is enabled to efficiently perform treatment in the living body lumen.

In the above-mentioned stent system, the stent is a drug eluting stent having a hollow tubular stent main body, and a coating layer provided at an outer surface of the stent main body and containing a drug, and, among an outer surface and the inner surface of the stent, the outer surface of the stent can be configured not to be provided with the modified surface. With this configuration, modification by surface treatment is not applied to the coating layer containing a drug of the stent. Therefore, adhesiveness between the balloon and the stent can be improved without any effect on the function of the coating layer containing a drug.

Furthermore, the invention is a method for manufacturing a stent system including a catheter having an inflatable and deflatable balloon, and a stent configured to expand along with inflation of the balloon characterized by including a provision step of providing the balloon and the stent, a surface treatment step of applying surface treatment to each of an outer surface of the balloon and an inner surface of the stent to modify the outer surface and the inner surface in such a way as to improve adhesiveness therebetween, and a mount step of mounting the stent with the surface-treated inner surface on an outer peripheral portion of the balloon with the surface-treated outer surface.

According to the above-mentioned method of the invention, since the outer surface of the balloon and the inner surface of the stent are modified by surface treatment applied thereto, the outer surface of the balloon and the inner surface of the stent interact with each other, so that adhesiveness between the outer surface of the balloon and the inner surface of the stent can be improved. Accordingly, when the stent is delivered to a lesion in the living body lumen, the stent can be prevented from becoming out of position with the balloon in the living body lumen, or the stent can be prevented from coming off there. Accordingly, the operator is enabled to efficiently perform treatment in the living body lumen.

In the above-mentioned method for manufacturing the stent system, the surface treatment step can include applying plasma treatment to the outer surface of the balloon and the inner surface of the stent under atmospheric pressure. With this, the outer surface of the balloon and the inner surface of the stent can be easily treated by plasma treatment under atmospheric pressure. Moreover, since a process for reduction in pressure and exposure to atmospheric pressure in a treatment chamber is not required unlike a case where plasma treatment is performed under the reduced-pressure atmosphere, the balloon and the stent can be combined with each other rapidly following the surface treatment with the treatment effect kept still high. Accordingly, high adhesiveness between the balloon and the stent can be effectively attained.

In the above-mentioned method for manufacturing the stent system, the surface treatment step can include applying surface treatment to the outer surface of the balloon in a deflated state in which the balloon is folded. This enables further reducing a time required to combine the balloon and the stent after plasma treatment.

In the above-mentioned method for manufacturing the stent system, the stent is a drug eluting stent having a hollow tubular stent main body, and a coating layer provided at an outer surface of the stent main body and containing a drug, and the surface treatment step can include modifying the inner surface of the stent by radiating energy from an axial direction of the stent in a state in which the outer surface of the stent is covered. This enables performing surface treatment on the inner surface of the stent without affecting the coating layer containing a drug.

In the above-mentioned method for manufacturing the stent system, the surface treatment step can include radiating the energy from both sides in an axial direction of the stent. This enables effectively performing surface treatment on the inner surface of the stent even in the case of the stent being long in the axial direction.

According to the stent system and the method for manufacturing the stent system of the invention, when the stent is delivered to a lesion in the living body lumen, the stent can be prevented from becoming out of position with the outer peripheral portion of the balloon.

### [Brief Description of the Drawings]

Fig. 1 is an overall schematic diagram of a stent system according to one embodiment of the invention.
Fig. 2 is an explanatory diagram of a method for performing surface treatment on the outer surface of a balloon by plasma treatment.
Fig. 3 is an explanatory diagram of another method for performing surface treatment on the outer surface of a balloon by plasma treatment.
Fig. 4 is an explanatory diagram of a method for performing surface treatment on the outer surface of a balloon by ultraviolet irradiation.
Fig. 5A is an explanatory diagram of a method for performing surface treatment on the inner surface of a stent by plasma treatment, and Fig. 5B is an explanatory diagram of a method for performing surface treatment on the inner surface of a long stent.
Fig. 6 is an explanatory diagram of another method for performing surface treatment on the inner surface of a stent.
Fig. 7 is a graph illustrating results of measurements of the retention strength of the stent (the degree of unlikelihood of becoming out of position of the stent) with respect to the balloon in Comparative Examples 1 to 3 and an Example of the invention.

### [Mode for Carrying Out the Invention]

Hereinafter, a stent system and a method for manufacturing the stent system according to the invention will be described by using preferred embodiments with reference to the accompanying drawings.

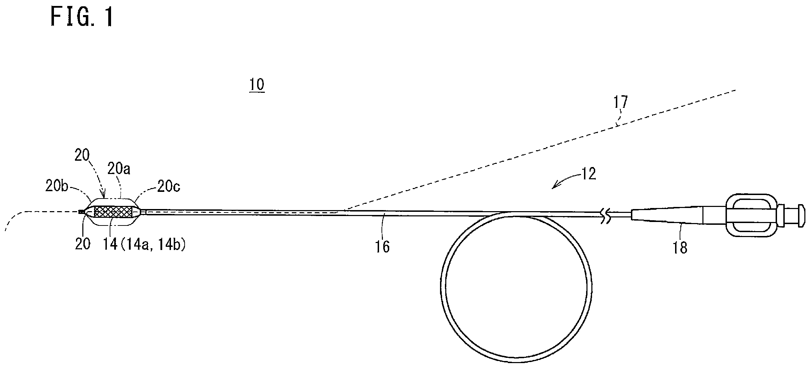

Fig. 1 is an overall schematic diagram of a stent system 10 according to one embodiment of the invention. The stent system 10 is used as a stent delivery, which is configured to be inserted through into a biological organ, for example, the coronary artery, and to expand a stent 14 provided at the distal end thereof at a lesion (stenosed site) to widen the lesion and then to place the stent 14 at the lesion. The invention is also applicable to uses other than for the coronary artery, for example, for the purpose of improving a lesion formed in a biological organ, such as another blood vessel, bile duct, trachea, esophagus, urethra, or other organs.

The stent system 10 includes a catheter 12 and stent 14, which is attached to the vicinity of the distal portion of the catheter 12. The catheter 12 has a thin and long shaft 16, a hub 18 provided at the proximal side of the shaft 16, and a balloon 20 provided at the distal side of the shaft 16, and is a delivery catheter configured to deliver the stent 14 to a target site in the living body lumen.

The shaft 16 is formed in a tubular shape and made from, for example, a resin having high sliding property, and has adequate flexibility and adequate strength so as to enable the operator to smoothly insert the shaft 16 through into a biological organ, such as a blood vessel, while grasping and manipulating the proximal side thereof. The material used to make the shaft 16 can be selected from among materials described below as examples of the material used to make the balloon 20, or can be another material.

Although the detailed illustration is omitted, a guide wire lumen, into which a guide wire 17 used to guide the stent system 10 to a lesion of the coronary artery is inserted through, and a balloon lumen, which is used to guide an inflation fluid into the balloon 20, are formed in the shaft 16.

In Fig. 1, the catheter 12 is configured as what is called a "rapid exchange type" catheter, which is provided with an opening portion, via which the guide wire 17 exits, at the halfway portion in the longitudinal direction of the shaft 16. In another embodiment, the catheter 12 can be configured as an "over the wire type" catheter, in which the guide wire lumen is formed over the entire length of the catheter 12 and the guide wire 17 exits from the proximal end of the hub 18.

The balloon 20 is able to inflate and deflate according to supply and drainage of an inflation fluid with respect to the balloon 20, and is bonded to the vicinity of the distal portion of the shaft 16 in a liquid-tight manner with respect to the shaft 16. The balloon 20 is folded in a deflated state. The balloon 20 being folded in a deflated state, as mentioned herein, refers to the balloon 20 being deflated and one or more portions of the outer surface of the balloon 20 being folded in a circumferential direction thereof.

In Fig. 1, the balloon 20 in an inflated state is indicated by a virtual line. The balloon 20 includes a tube portion 20a, which is able to inflate into a tubular shape (cylindrical shape) the outer diameter of which is approximately constant in the axial direction, a distal taper portion 20b, the outer diameter of which is gradually reduced at the distal side of the tube portion 20a, and a proximal taper portion 20c, the outer diameter of which is gradually reduced at the proximal side of the tube portion 20a.

Such a balloon 20 needs to have adequate flexibility, and also needs to have such a degree of strength as to be able to securely widen the stenosed site. More specifically, it is made from an organic polymeric material. Accordingly, for example, it can be formed of a polymer material, such as polyolefin (for example, polyethylene, polypropylene, polybutene, ethylenepropylene copolymer, ethylene-vinyl acetate copolymer, ionomer, or a mixture of two or more of these materials), polyvinyl chloride, polyamide, polyamide elastomer, polyurethane, polyurethane elastomer, polyimide, and fluorine resin, or a mixture of some of these materials, or a multi-layer tube or the like made from two or more of the above-mentioned polymer materials.

The stent 14 is what is called a balloon-expandable stent, which expands while plastically deforming due to a dilation force of the balloon 20, and is mounted on the tube portion 20a of the balloon 20.

The configuration of the stent 14 includes, for example, a configuration which has a mesh-like side peripheral wall provided with multiple side holes and is formed of a tube (cylinder) as an overall shape, and a configuration which is formed of a tube (cylinder) as an overall shape by connecting, in the axial direction, multiple circular bodies, each of which extends in a wavelike fashion in the circumferential direction. The distal end and proximal end of the stent 14 each can be provided with a radiopaque marker made from, for example, a radiopaque material.

The material used to make a framework (strut) of the stent 14 can include, for example, metal having biocompatibility, and a biodegradable polymer having bioabsorbability.

Examples of the metal having biocompatibility include an iron-base alloy, such as stainless steel, tantalum (a tantalum alloy), platinum (a platinum alloy), gold (a gold alloy), a cobalt-base alloy, a cobalt chrome alloy, a titanium alloy, and a niobium alloy.

The biodegradable polymer is a polymer which gradually biodegrades after the stent 14 is placed at the lesion and has no adverse influence on the living body of a human or animal. The biodegradable polymer is not specifically limited, but is desirably the one having high biological stability, and, for example, is desirably at least one polymer selected from the group consisting of polylactic acid, polyglycolic acid, a copolymer of lactic acid and glycolic acid, polycaprolactone, polyhydroxybutyrate, polyhydroxybutyrate valeric acid, polymalic acid, poly-α-amino acid, polyorthoester, cellulose, collagen, laminin, heparan sulfate, fibronectin, vitronectin, chondroitin sulfate, hyaluronic acid, cinnamic acid, and a cinnamic acid derivative, a copolymer obtained by copolymerization of any of monomers constituting the polymers, or a mixture of the polymer and the copolymer.

The stent 14 can be the one including a stent main body 14a (strut) constituting a framework, and a coating layer 14b (a drug-containing polymer layer) containing a drug provided on the outer surface of the stent main body 14a. The coating layer 14b can be formed on the entirety of the outer surface of the stent main body 14a, or can be formed on a part of the outer surface of the stent main body 14a.

The drug contained in the coating layer 14b is a biological physiological active substance, and, after placement of the stent 14 in the living body lumen, has the effect of preventing restenosis by gradually eluting at a region in which the stenosed site has been treated. Examples of such a drug include anticancer drug, immunosuppressive drug, antibiotic, antirheumatic, antithrombogenic drug, antihyperlipidemic drug, angiotensin-converting enzyme inhibitor, calcium antagonist agent, integrin inhibitor, anti-allergic drug, antioxidant agent, GpIIb/IIIa inhibitor, retinoid, flavonoid, carotenoid, lipid improving drug, DNA synthesis inhibitor, tyrosine kinase inhibitor, antiplatelet drug, vascular smooth muscle growth inhibitor, anti-inflammatory drug, a tissue-derived biomaterial, interferon, and NO production promoting substance.

In the catheter 12 according to the present embodiment, the outer surface of the balloon 20 has a surface modified by surface treatment in such a way as to have improved adhesiveness with the inner surface of the stent 14. Moreover, the inner surface of the stent 14 has a surface modified by surface treatment in such a way as to have improved adhesiveness with the outer surface of the balloon 20.

More specifically, surface treatment is applied to at least the outer surface of the tube portion 20a in the outer surface of the balloon 20. In this case, the range at which surface treatment is applied to the outer surface of the tube portion 20a can be the entirety of the outer surface of the tube portion 20a, can be a part of that outer surface as viewed in the axial direction, can be a part of that outer surface as viewed in the circumferential direction, or can be a part of that outer surface as viewed in the axial direction and the circumferential direction. The range at which surface treatment is applied to the inner surface of the stent 14 can be the entirety of that inner surface, can be a part of that inner surface as viewed in the axial direction, can be a part of that inner surface as viewed in the circumferential direction, or can be a part of that inner surface as viewed in the axial direction and the circumferential direction.

Examples of the surface treatment include plasma treatment, ultraviolet irradiation, and corona discharge. In particular, in terms of easiness of surface treatment, plasma treatment under atmospheric pressure is desirable. More desirably, it is plasma treatment in the presence of helium gas.

Applying such surface treatment to the outer surface of the balloon 20 causes all or at least one of the effects of (a) addition of, for example, a carboxyl group, a hydroxyl group, or a amino group to the surface of a polar group, (b) removal of fine organic dirt adhering to the surface, and (c) application of roughness to the surface.

In the case of the stent 14 being made from metal, applying surface treatment to the metal surface (metal-exposed surface) of the inner surface of the stent 14 causes all or at least one of the effects of (d) activation of water molecules hydrated with an oxide layer or removal of bound water, and (e) cleaning of fine organic dirt adhering to the surface.

In the case of the coating layer 14b made from a polymer being provided on the inner surface of the stent 14, applying surface treatment to the inner surface (a portion corresponding to the coating layer 14b) of the stent 14 causes all or at least one of the above-mentioned effects (a) to (c) on the inner surface of the stent 14.

In the plasma treatment, plasma can be generated in a vacuum, or plasma can be generated under atmospheric pressure.

The ultraviolet irradiation is desirably short-wavelength ultraviolet irradiation, which allows irradiation with high energy. The wavelength of short-wavelength ultraviolet is of the order of, for example, 180 nm to 310 nm.

The stent system 10 according to the present embodiment is basically configured as described above, and the function and effect thereof are described as follows.

According to the stent system 10, since both the outer surface of the balloon 20 and the inner surface of the stent 14 are modified to be activated and the modified surfaces are in contact with each other, the modified surfaces interact with each other, so that adhesiveness between the outer surface of the balloon 20 and the inner surface of the stent 14 can be effectively improved. In other words, the coefficient of friction between the outer surface of the balloon 20 and the inner surface of the stent 14 can be improved as compared with a case where the surfaces are not subjected to property modification. Accordingly, when the stent system 10 is used (when the stent 14 is delivered to a lesion in the living body lumen), the stent 14 can be prevented from becoming out of position with the balloon 20 in the living body lumen, or the stent 14 can be prevented from coming off there. Accordingly, the operator is enabled to efficiently perform treatment in the living body lumen.

Furthermore, in the case where the stent 14 is a drug eluting stent, among the outer surface and the inner surface of the stent 14, the outer surface is not provided with the modified surface. With this configuration, property modification by surface treatment is not applied to the coating layer 14b containing a drug of the stent 14. Therefore, adhesiveness between the balloon 20 and the stent 14 can be improved without any effect on the function of the coating layer 14b containing a drug.

Moreover, since adhesiveness between metal and an organic polymeric material is low, the surface treatment in the invention is effective for the case of attempting to increase adhesiveness between metal and an inelastic organic polymeric material. In other words, the surface treatment in the invention is particularly effective for the case where the stent 14 is made from metal and the balloon 20 is made from an organic polymeric material. Furthermore, the organic polymeric material subjected to surface treatment has an increased adhesiveness with organic metal since a polar group is introduced into a portion subjected to the surface treatment. Therefore, it is desirable that the inner side of the stent 14 be provided with an oxide layer formed thereon.

Next, a method for manufacturing the stent system 10 configured as described above is described.

The method for manufacturing the stent system 10 includes a provision step of providing the balloon 20 and the stent 14, a surface treatment step of applying surface treatment to each of the outer surface of the balloon 20 and the inner surface of the stent 14 to modify the outer surface and the inner surface in such a way as to improve adhesiveness therebetween, and a mount step of mounting the stent 14 with the surface-treated inner surface on an outer peripheral portion of the balloon 20 with the surface-treated outer surface.

More specifically, the above-mentioned provision step includes a balloon provision step of providing the balloon 20 (the one with the outer surface not yet subjected to surface treatment), and a stent provision step of providing the stent 14 (the one with the inner surface not yet subjected to surface treatment).

The balloon provision step can include providing a catheter 12 that is composed of a combination of the shaft 16, the hub 18, and the balloon 20. Moreover, the catheter 12 as used herein can be the one that is in a deflated state in which the balloon 20 is folded.

In the case where the stent 14 is a drug eluting stent, the stent provision step includes providing a stent 14 in which the coating layer 14b is formed on the outer surface of the stent main body 14a.

The above-mentioned surface treatment step includes applying surface treatment to the outer surface of the balloon 20 and the inner surface of the stent 14 provided in the provision step. The following are some examples of a method for surface treatment, but the present invention is not limited to these examples.

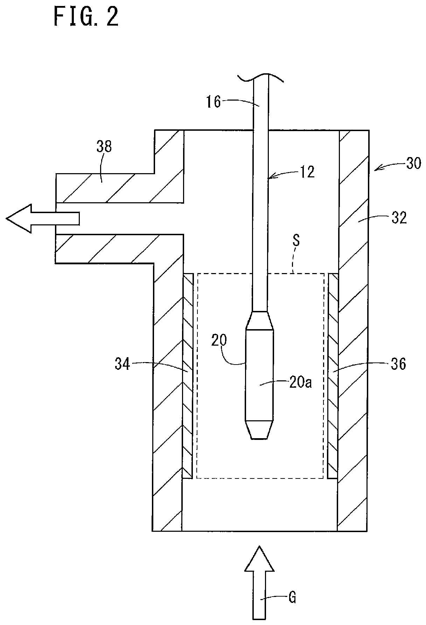

Fig. 2 is an explanatory diagram of a method for performing surface treatment on the outer surface of the balloon 20 by plasma treatment (energy irradiation) under atmospheric pressure. In the case of a plasma generation device 30 illustrated in Fig. 2, a first electrode 34 and a second electrode 36 are arranged inside a plasma discharge tube 32 both ends of which in the axial direction are opened, and a process gas G is set to be continuously or intermittently supplied to the plasma discharge tube 32.

A high-frequency power source (not illustrated) is connected to the first electrode 34 and the second electrode 36, and, when a high-frequency voltage is applied to the first electrode 34 and the second electrode 36, the process gas G is converted into a plasma by electric discharge. With this, a plasma region S is formed inside the plasma discharge tube 32. The process gas G is continuously discharged via an exhaust portion 38. Examples of the process gas G include a noble gas, such as helium and argon.

As illustrated in Fig. 2, when the balloon 20 is inserted into the plasma discharge tube 32 in which a plasma is generated, the outer surface of the balloon 20 is exposed to the plasma. At this time, the position of the balloon 20 can be adjusted such that the entirety of the tube portion 20a of the balloon 20 is located within the plasma region S. Plasma treatment on the outer surface of the balloon 20 can be performed by exposing the outer surface of the balloon 20 to a plasma for a predetermined time.

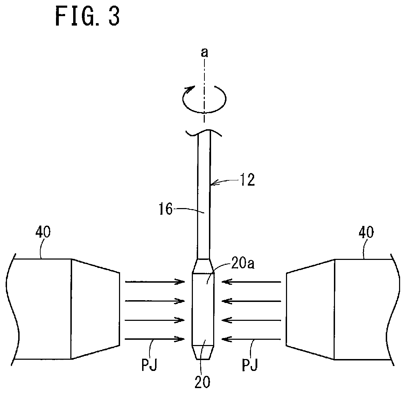

Fig. 3 is an explanatory diagram of another method for performing surface treatment on the outer surface of the balloon 20 by plasma treatment (energy irradiation) under atmospheric pressure. In the method illustrated in Fig. 3, at least one plasma nozzle 40 that emits a plasma (a plasma jet PJ) is used to irradiate the outer surface of the balloon 20 with the emitted plasma. In this case, radiating the plasma while rotating the balloon 20 and the plasma nozzle 40 relative to each other around the axial line "a" of the catheter 12 enables irradiating the entire circumference of the outer surface of the balloon 20 with the plasma. The relative rotation of the balloon 20 and the plasma nozzle 40 can be performed by rotating the catheter 12 around the axial line "a" or by rotating the plasma nozzle 40 around the axial line "a" of the catheter 12.

Furthermore, as illustrated in Fig. 3, emitting plasmas from a plurality of (in Fig. 3, two) plasma nozzles 40, which are arranged around the balloon 20, toward the outer surface of the balloon 20 while rotating the plurality of plasma nozzles 40 and the balloon 20 relative to each other enables efficiently irradiating the entire circumference of the outer surface of the balloon 20 with the plasmas.

Moreover, radiating a plasma from the plasma nozzle 40 toward the outer surface of the balloon 20 while displacing the plasma nozzle 40 and the balloon 20 relative to each other along the axial direction of the catheter 12 can be employed. This enables efficiently irradiating the entire length of the tube portion 20a of the balloon 20 with a plasma even in a case where the exit port diameter of the plasma nozzle 40 is less than the length of the tube portion 20a.

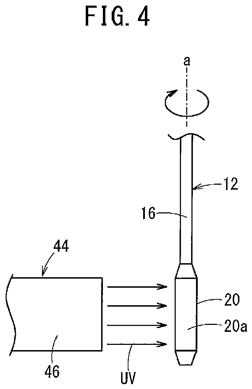

Fig. 4 is an explanatory diagram of a method for performing surface treatment on the outer surface of the balloon 20 by ultraviolet irradiation (energy irradiation) under atmospheric pressure. In the method illustrated in Fig. 4, an ultraviolet irradiation device 44 is used to irradiate the outer surface of the balloon 20 with ultraviolet (UV). The UV source of the ultraviolet irradiation device 44 includes a low-pressure mercury lamp (wavelengths of 182 nm and 254 nm) and an excimer lamp (wavelengths of 126 nm to 308 nm).

In this case, radiating ultraviolet while rotating the balloon 20 and an output portion 46 of the ultraviolet irradiation device 44 relative to each other around the axial line "a" of the catheter 12 enables irradiating the entire circumference of the outer surface of the balloon 20 with ultraviolet. The relative rotation of the balloon 20 and the output portion 46 can be performed by rotating the catheter 12 around the axial line "a" or by rotating the output portion 46 around the axial line "a" of the catheter 12.

Furthermore, although not illustrated, radiating ultraviolet from a plurality of output portions 46, which are arranged around the balloon 20, toward the outer surface of the balloon 20 while rotating the plurality of output portions 46 and the balloon 20 relative to each other enables efficiently irradiating the entire circumference of the outer surface of the balloon 20 with ultraviolet.

Moreover, radiating ultraviolet from the output portion 46 toward the outer surface of the balloon 20 while displacing the output portion 46 and the balloon 20 relative to each other along the axial direction of the catheter 12 can be employed. This enables efficiently irradiating the entire length of the tube portion 20a of the balloon 20 with ultraviolet even in a case where the output width of ultraviolet corresponding to the size of the output portion 46 is less than the length of the tube portion 20a.

Furthermore, although not illustrated, it is desirable that the surface treatment of the balloon 20 be performed in a state in which the balloon 20 is folded (in a deflated state). With this, in the state in which the balloon 20 is folded, adhesiveness between the balloon 20 and the stent 14 can be increased. Moreover, in a state in which the balloon 20 is inflated, adhesiveness between the balloon 20 and the stent 14 can be decreased. More specifically, performing surface treatment in the state in which the balloon 20 is folded enables forming a surface-treated portion and a non-surface-treated portion on the outer surface of the balloon 20 in the inflated state. This is because, in the state in which the balloon 20 is folded, surface treatment of the balloon 20 is not applied to a folded portion of the balloon 20. Here, a non-surface-treated portion of the outer surface of the balloon 20 is weak in interaction with the stent 14 and is low in adhesiveness. Therefore, in the state in which the balloon 20 is inflated, adhesiveness between the balloon 20 and the stent 14 is decreased. Accordingly, during delivery in the living body lumen, the stent 14 can be prevented from coming off the balloon 20. Furthermore, when the stent 14 is placed in the living body lumen, since the stent 14 is easily detached from the balloon 20, placement of the stent 14 can be easily performed.

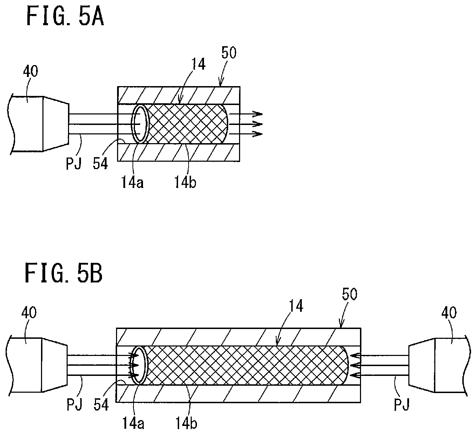

Fig. 5A is an explanatory diagram of a method for performing surface treatment on the inner surface of the stent 14 by plasma treatment (energy irradiation) under atmospheric pressure. The method illustrated in Fig. 5A is particularly useful for a case where the stent 14 is a drug eluting stent. In the case of the drug eluting stent, the physical property of the coating layer 14b provided on the outer surface of the stent main body 14a fulfills an important role for treatment effect. Accordingly, it is desirable that surface treatment be set not to cause an effect, such as thermal decomposition, to a drug and a polymer constituting the coating layer 14b.

Therefore, in the method illustrated in Fig. 5A, the stent 14 is arranged inside a hollow tubular cover member 50, both ends of which are opened, and, while the outer surface (the coating layer 14b) of the stent 14 is covered, a plasma (a plasma jet PJ) is radiated toward a hollow portion of the stent 14 from the axial direction of the stent 14. In this case, the cross-sectional shape of an inner peripheral portion 54 of the cover member 50 perpendicular to the axial direction is a circular shape similar to that of the stent 14. According to such a method, surface treatment can be applied to a surface (mainly, the inner surface) other than the outer surface of the stent 14 without any influence on the coating layer 14b, which contains a drug.

In the case of a stent 14 having a length of 20 mm or more in the axial direction, plasmas (plasma jets PJ) can be radiated toward a hollow portion of the stent 14 from both sides in the axial direction of the stent 14, as illustrated in Fig. 5B. This enables effectively performing surface treatment on the inner surface of the stent 14 even in the case of the stent 14 being long in the axial direction.

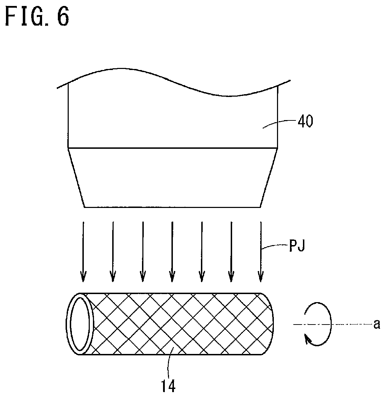

Fig. 6 is an explanatory diagram of another method for performing surface treatment on the inner surface of the stent 14 by energy irradiation under atmospheric pressure. In the method illustrated in Fig. 6, a plasma (a plasma jet PJ) is radiated toward the outer surface of stent 14 from a plasma nozzle 40, which is arranged opposite to the outer surface of the stent 14, so that the outer surface of the stent 14 is irradiated with the plasma. With this, since the plasma passes through the meshes (side holes) of the stent 14 and reaches the inner surface of the stent 14, plasma treatment can be applied to the inner surface of the stent 14.

In this case, radiating the plasma while rotating the stent 14 and the plasma nozzle 40 relative to each other around the axial line "a" of the stent 14 enables irradiating the entire circumference of the inner surface of the stent 14 with the plasma. For example, as illustrated in Fig. 6, the stent 14 can be rotated around the axial line "a" of the stent 14. Moreover, instead of plasma treatment, for example, ultraviolet irradiation or corona discharge treatment can be applied.

According to the method illustrated in Fig. 6, while surface treatment is applied to not only the inner surface but also the outer surface of the stent 14, in the case where the stent 14 is not a drug eluting stent, there is no problem with the outer surface of the stent 14 being subjected to surface treatment.

After the balloon 20 with the outer surface surface-treated (the catheter 12 provided with the balloon 20) and the stent 14 with the inner surface surface-treated are obtained as mentioned above, then, a mount step of combining the balloon 20 and the stent 14 is performed.

The mount step includes mounting the stent 14 on the outer peripheral portion of the balloon 20, which is folded into a deflated state. More specifically, in a state in which the balloon 20 in the deflated state and the stent 14 are arranged in a concentric fashion and the tube portion 20a of the balloon 20 and the stent 14 are superposed in the axial direction, the stent 14 is contracted in the radial direction, so that the stent 14 is mounted on the outer peripheral portion of the balloon 20. With this, the stent 14 decreases in diameter while plastically deforming, so that the outer surface of the balloon 20 and the inner surface of the stent 14 adhere tightly to each other.

After the completion of the mount step, predetermined packing and sterilization are performed, so that the stent system 10 is completed as a product.

According to the above-described method for manufacturing the stent system 10, since the outer surface of the balloon 20 and the inner surface of the stent 14 are modified by surface treatment applied thereto, the outer surface of the balloon 20 and the inner surface of the stent 14 interact with each other, so that adhesiveness between the outer surface of the balloon 20 and the inner surface of the stent 14 can be improved. Accordingly, when the stent 14 is delivered to a lesion in the living body lumen, the stent 14 can be prevented from becoming out of position with the balloon 20 in the living body lumen, or the stent 14 can be prevented from coming off there. Accordingly, the operator is enabled to efficiently perform treatment in the living body lumen.

As described above, in the surface treatment step, if plasma treatment is applied to the outer surface of the balloon 20 and the inner surface of the stent 14 under atmospheric pressure, the outer surface of the balloon 20 and the inner surface of the stent 14 can be easily treated.

Usually, the effect of surface treatment tends to decrease with time. Accordingly, it is desirable that the mount step be performed rapidly following the surface treatment step. In other words, it is desirable that the mount step be performed when the effect of surface treatment obtained in the surface treatment step is still high.

In the case of the present embodiment, since a process for reduction in pressure and exposure to atmospheric pressure in a treatment chamber is not required unlike a case where plasma treatment is performed under the reduced-pressure atmosphere, the balloon 20 and the stent 14 can be combined with each other rapidly following the surface treatment with the treatment effect kept still high. Accordingly, high adhesiveness between the balloon 20 and the stent 14 can be effectively attained. In this case, it is desirable that the mount step be performed within, for example, 60 minutes, desirably 30 minutes, of the completion of the surface treatment on the outer surface of the balloon 20 and the inner surface of the stent 14 under atmospheric pressure.

Furthermore, in the surface treatment step, since surface treatment is applied to the outer surface of the balloon 20 in a deflated state in which the balloon 20 is folded, a time required to combine the balloon 20 and the stent 14 after plasma treatment can be further reduced.

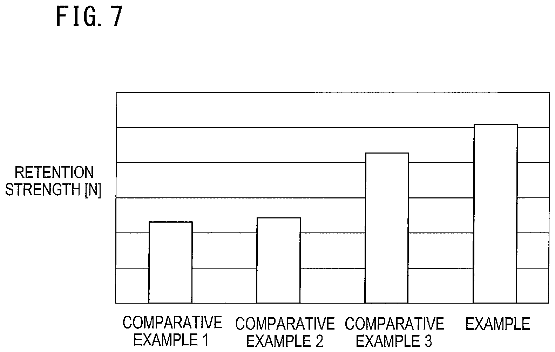

Fig. 7 is a graph illustrating results of measurements of the retention strength of the stent (the degree of unlikelihood of becoming out of position of the stent) with respect to the balloon in Comparative Examples 1 to 3 and an Example of the invention.

Referring to Fig. 7, in the Comparative Example 1, surface treatment, such as plasma treatment, was applied to neither the outer surface of the balloon nor the inner surface of the stent. In the Comparative Example 2, while surface treatment was applied to the inner surface of the stent, surface treatment was not applied to the outer surface of the balloon. In the Comparative Example 3, while surface treatment was applied to the outer surface of the balloon, surface treatment was not applied to the inner surface of the stent. In the Example of the invention, surface treatment was applied to both the outer surface of the balloon 20 and the inner surface of the stent 14.

As understood from Fig. 7, in the case of the Comparative Example 2, in which surface treatment was applied to only the inner surface of the stent, there was little difference in retention strength as compared with the Comparative Example 1, in which no surface treatment was applied. On the other hand, in the case of the Example of the invention, in which surface treatment was also applied to the inner surface of the stent 14 in addition to the outer surface of the balloon 20, a distinctly high retention strength was obtained as compared with the Comparative Example 3, in which surface treatment was applied to only the outer surface of the balloon. From this, it is understood that, while surface treatment on only the inner surface of the stent 14 hardly contributes to the improvement in retention strength, applying surface treatment, such as plasma treatment, to both the outer surface of the balloon 20 and the inner surface of the stent 14 causes surface treatment on the inner surface of the stent 14 to greatly contribute to the improvement in retention strength.

Therefore, according to the invention, adhesiveness between the outer surface of the balloon 20 and the inner surface of the stent 14 can be effectively improved.

While the invention has been described above with reference to preferred embodiments thereof, the invention is not limited to the above-described embodiments, but can be naturally modified or altered in various manners without departing from the gist of the invention.

## Claims

1. A stent system (10) **characterized by** comprising:
a catheter (12) having an inflatable and deflatable balloon (20); and
a stent (14) mounted on an outer peripheral portion of the balloon (20) and configured to expand along with inflation of the balloon (20),
wherein an outer surface of the balloon (20) and an inner surface of the stent (14) have respective modified surfaces, and adhesiveness between the outer surface and the inner surface is improved by the modified surfaces being in contact with each other.

2. The stent system (10) according to claim 1,
**characterized in that** the stent (14) is a drug eluting stent (14) having a hollow tubular stent main body (14a), and a coating layer (14b) provided at an outer surface of the stent main body (14a) and containing a drug, and
among an outer surface and the inner surface of the stent (14), the outer surface of the stent is not provided with the modified surface.

3. A method for manufacturing a stent system (10) including a catheter (12) having an inflatable and deflatable balloon (20), and a stent (14) configured to expand along with inflation of the balloon (20), the method for manufacturing the stent system (10) **characterized by** comprising:
a provision step of providing the balloon (20) and the stent (14);
a surface treatment step of applying surface treatment to each of an outer surface of the balloon (20) and an inner surface of the stent (14) to modify the outer surface and the inner surface in such a way as to improve adhesiveness therebetween; and
a mount step of mounting the stent (14) with the surface-treated inner surface on an outer peripheral portion of the balloon (20) with the surface-treated outer surface.

4. The method for manufacturing the stent system (10) according to claim 3,
**characterized in that** the surface treatment step includes applying plasma treatment to the outer surface of the balloon (20) and the inner surface of the stent (14) under atmospheric pressure.

5. The method for manufacturing the stent system (10) according to claim 4,
**characterized in that** the surface treatment step includes applying surface treatment to the outer surface of the balloon (20) in a deflated state in which the balloon (20) is folded.

6. The method for manufacturing the stent system (10) according to any one of claims 3 to 5,
**characterized in that** the stent (14) is a drug eluting stent (14) having a hollow tubular stent main body (14a), and a coating layer (14b) provided at an outer surface of the stent main body (14a) and containing a drug, and
the surface treatment step includes modifying the inner surface of the stent (14) by radiating energy from an axial direction of the stent (14) in a state in which the outer surface of the stent (14) is covered.

7. The method for manufacturing the stent system (10) according to claim 6,
**characterized in that** the surface treatment step includes radiating the energy from both sides in an axial direction of the stent (14).
